Europäisches Patentamt

European Patent Office

Office européen des brevets

(19)

(11) Veröffentlichungsnummer: **0 072 008**
**B1**

(12) **EUROPÄISCHE PATENTSCHRIFT**

(45) Veröffentlichungstag der Patentschrift:
**13.11.85**

(21) Anmeldenummer: **82107155.2**

(22) Anmeldetag: **07.08.82**

(51) Int. Cl.⁴: **C 07 C 25/02,** C 07 C 17/38,
C 07 C 17/00

(54) Verfahren zur Herstellung von p-Chlortoluol und/oder m-Chlortoluol.

(30) Priorität: **11.08.81 DE 3131682**

(43) Veröffentlichungstag der Anmeldung:
**16.02.83 Patentblatt 83/7**

(45) Bekanntmachung des Hinweises auf die Patenterteilung:
**13.11.85 Patentblatt 85/46**

(84) Benannte Vertragsstaaten:
**BE CH DE FR GB IT LI NL**

(56) Entgegenhaltungen:
**EP - A - 0 046 068**
**EP - A - 0 046 665**
**DE - A - 1 543 020**
**GB - A - 902 724**
**GB - A - 1 212 809**

(73) Patentinhaber: **HOECHST AKTIENGESELLSCHAFT,**
**Postfach 80 03 20, D-6230 Frankfurt am Main 80 (DE)**

(72) Erfinder: **Arpe, Hans-Jürgen, Prof. Dr.,**
**de-Ridder-Weg 10, D-6230 Frankfurt am Main 80 (DE)**
Erfinder: **Litterer, Heinz, Dr., Albert-Schweitzer-Allee 39,**
**D-6200 Wiesbaden (DE)**
Erfinder: **Mayer, Norbert, Dr., Heimchenweg 82,**
**D-6230 Frankfurt am Main 80 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Bekanntlich entstehen bei der Chlorierung von Toluol in Gegenwart von Katalysatoren die kernchlorierten isomeren o-, m- und p-Monochlortoluole in einem je nach eingesetztem Katalysatortyp und gewählten Reaktionsbedingungen wechselnden Verhältnis. So führt die Chlorierung von Toluol beispielsweise bei 50° C in Gegenwart von $FeCl_3$ als Katalysator bis zu einer Dichte von 1,043 g/cm³ (20° C) zu einem Produkt aus 19 Gew.-% Toluol, 49 Gew.-% o-Chlortoluol, 2 Gew.-% m-Chlortoluol und 25,5 Gew.-% p-Chlortoluol. Daneben bilden sich zusätzlich 4,5% Dichlortoluol (Ullmanns Encycl. der techn. Chemie [1975], Band 9, Seite 512). Die Verwendung von $TiCl_4$, $SnCl_4$, $WCl_6$ oder $ZrCl_4$ als Katalysatoren bei einer Chlorierungstemperatur von 10—30° C erhöht den o-Chlortoluolanteil auf 75 Gew.-% (US-PS 3 000 975), während der Einsatz von Dischwefeldichlorid und den genannten Metallchloriden oder von Schwefel und $SbCl_3$ das Isomerenverhältnis zugunsten des p-Chlortoluols verschiebt (US-PS 1 946 040). In keinem Fall gelingt es, durch geeignete Wahl des Katalysators oder der Reaktionsbedingungen zu einem einheitlichen Isomeren zu gelangen. Daher ist bei allen diesen Verfahren eine nachfolgende Auftrennung des Reaktionsgemisches in die reinen Isomeren notwendig, welche üblicherweise zweistufig durchgeführt wird, wobei in einem ersten, apparativ- und energieaufwendigen Aufarbeitungsschritt o-Chlortoluol (Sdp. 158,5° C) destillativ, z. B. in einer Glockenbodenkolonne mit über 200 theoretischen Böden, als Kopfprodukt von einem Sumpf aus m- und p-Chlortoluol abgetrennt wird. Wegen der geringen Siedepunktdifferenz dieser Isomeren (161,2° C bzw. 161,5° C) kann ihr Gemisch nicht mehr destillativ aufgetrennt werden. Reines p-Chlortoluol (Fp 7,6° C) muß deshalb in einem zweiten Schritt durch Kristallisation vom tiefer schmelzenden m-Isomeren (Fp −48,7° C) abgetrennt werden.

Der Erfindung lag die Aufgabe zugrunde, diese umständliche Auftrennung zu vereinfachen. Ein weiteres Ziel der Erfindung bestand darin, die Ausbeute an dem technisch wichtigen p-Chlortoluol zu erhöhen und darüber hinaus — bei Bedarf — eine einfache Herstellung und Isolierung von m-Chlortoluol zu ermöglichen.

Das erfindungsgemäße Verfahren zur Herstellung von p-Chlortoluol und/oder m-Chlortoluol durch Kernchlorierung von Toluol ist gekennzeichnet durch Verwendung der beiden folgenden Stufen a) und b) bei der Weiterverarbeitung des aus einem Isomerengemisch bestehenden Toluol-Chlorierungsproduktes:

a) Isolation von p-Chlortoluol aus einem Gemisch von o-, m- und p-Chlortoluol durch selektive Adsorption des p-Chlortoluols an einem mittel- oder großporigen Zeolith und anschließende Desorption des p-Chlortoluols,

b) Behandlung eines an o-Chlortoluol reichen Isomerengemisches mit einem Zeolith vom Pentasil-, Mordenit- oder Faujasit-Typ als Isomerisierungskatalysator,

wobei man zur
ausschließlichen Herstellung von p-Chlortoluol zunächst aus dem Toluol-Chlorierungsprodukt in Stufe a) p-Chlortoluol isoliert und ausschleust, dann das verbleibende Isomerengemisch in Stufe b) behandelt, um seinen Gehalt an m- und p-Isomeren zu erhöhen, und dann in Stufe a) zurückführt, und
zur Herstellung von m-Chlortoluol, und gegebenenfalls zusätzlich von p-Chlortoluol, zunächst das Toluol-Chlorierungsprodukt in Stufe b) behandelt, um seinen Gehalt an m- und gegebenenfalls an p-Isomerem zu erhöhen, dann in Stufe a) p-Chlortoluol isoliert und dieses in Stufe b) zurückführt oder gegebenenfalls ausschleust und das verbleibende, im wesentlichen aus o- und m-Isomeren bestehende Gemisch destillativ oder adsorptiv trennt und dann das o-Chlortoluol in Stufe b) zurückführt und das m-Chlortoluol ausschleust.

Aus GB-A-902 724 ist bereits bekannt, daß man Isomerengemische von Chlortoluolen durch selektive Adsorption an Zeolithen trennen kann. Eine Isomerisation wird jedoch nicht durchgeführt, insbesondere nicht mit Zeolithen. Aus GB-A-1 212 809 ist bereits eine Isomerisation von Chlortoluolen bekannt, jedoch wird sie nicht mit einem Zeolith durchgeführt, sondern mit einem Friedel-Crafts-Katalysator, nämlich $BF_3 \cdot HF$.

Geeignete mittel- oder großporige Zeolithe (Porendurchmesser >0,46 nm) für die in Schritt a) vorgenommene selektive Adsorption des p-Chlortoluols aus dem Gemisch der Isomeren sind beispielsweise modifizierte Pentasile sowie modifizierte X- und Y-Zeolithe. Eine geeignete Modifizierung wird durch Ionenaustausch und/oder Imprägnierung mit ein- oder mehrwertigen Kationen erreicht. Als Kationen kommen in Frage: $H^+$, $NH_4^+$, $Na^+$, $K^+$, $Ca^{2+}$, $Sr^{2+}$, $Ba^{2+}$, $Zr^{4+}$, $Co^{2+}$. Besonders geeignet sind mit $K^+$, $Ba^{2+}$ oder $K^+/Ba^{2+}$ ausgetauschte Y-Zeolithe. Durch zusätzlichen Einbau von Protonen (über die $NH_4^+$-Form) wird an hochausgetauschten Kalium- oder Barium-Zeolithen des Y-Typs ein Maximum der p-Chlortoluol-Selektivität bei einem Maximum der dynamischen Adsorptionskapazität erreicht.

Eine andere Modifizierung zur Verbesserung der p-Chlortoluol-Selektivität von Pentasilen ist ein gezieltes Precoking, welches anstelle von Ionenaustausch bzw. Imprägnierung oder auch zusätzlich zu diesen Maßnahmen durchgeführt werden kann. Hierzu werden Kohlenwasserstoffe, bevorzugt aromatische wie Toluol, bei Temperaturen von 500 bis 800° C mit dem Pentasil kurzzeitig in Kontakt gebracht.

Um die Zeolithe in eine einsatzfähige Form zu bringen, sind im allgemeinen Bindermaterialien erforderlich, z. B. die Oxide, Hydroxide oder Hydroxychloride des Aluminiums oder die Oxide oder Hydroxide des Siliciums.

Die Adsorption in Schritt a) wird bei Temperaturen von 150 bis 300°C, vorzugsweise bei 180 bis 280°C, und bei einem Druck von 1 bis 50 bar, vorzugsweise bei 5 bis 25 bar durchgeführt. Die anschließende Desorption wird im allgemeinen mit Hilfe eines Kohlenwasserstoffes oder mit Hilfe von Wasserdampf, Ammoniak, Stickstoff, Wasserstoff oder einem anderen Inertgas durchgeführt, und zwar vorzugsweise unter erhöhter Temperatur und herabgesetztem Druck (relativ zur Adsorption).

Zur Isomerisierung in Stufe b) verwendet man synthetische Zeolithe vom Pentasil-Typ wie ZSM-5, ZSM-8 oder ZSM-11 — welche beispielsweise nach GB-PS 1 567 948 zugänglich sind — sowie natürliche oder synthetische Zeolithe vom Mordenit- oder Faujasit-Typ. Das Si/Al-Verhältnis der Pentasile liegt vorzugsweise bei etwa 25 bis 2000, das der Mordenite bei vorzugsweise 5 bis 100. Bei Pentasilen oder Mordeniten mit einem höheren Aluminiumgehalt kann durch Behandlung mit Mineralsäuren, organischen Säuren oder chelatisierenden Substanzen eine teilweise Entfernung des Gerüstaluminiums erreicht werden, was eine Steigerung der Wirksamkeit zur Folge hat. Die genannten Zeolithe werden für den technischen Einsatz mit Hilfe von Bindern in Strangform gebracht, wobei die Wahl des Binders die Selektivität und Lebensdauer beeinflußt.

Als Bindermaterialien sind vor allem die Oxide, Hydroxide oder Hydroxychloride des Aluminiums und die Oxide bzw. Hydroxide des Siliciums sowie Tone und Claymaterialien geeignet. Die Zeolithe werden durch Ionenaustausch in ihre katalytisch aktiven Formen überführt. Besonders geeignete Kationen sind $H^+$, $NH_4^+$, $Mg^{2+}$, $Ca^{2+}$, die Selten-Erdmetallionen sowie Kombinationen dieser Elemente.

Die Zeolith-Katalysatoren werden außerdem in der üblichen Weise durch Kalzinieren aktiviert. Gelegentlich ist es vorteilhaft, Ionenaustausch und Kalzinieren mehrmals zu wiederholen. Die Kalzinierung wird vorzugsweise bei 350 bis 700°C durchgeführt. Zur besseren Stabilisierung ist es manchmal vorteilhaft, die Kalzinierung in Gegenwart von Wasserdampf, Ammoniak oder deren Mischungen bei Temperaturen zwischen 600 und 900°C durchzuführen.

Die Isomerisierung kann sowohl in der Gasphase als auch in der Flüssigphase durchgeführt werden. Geeignete Prozeßbedingungen für die Flüssigphasen-Isomerisierung sind Temperaturen von 200 bis 350°C, vorzugsweise 200 bis 300°C, und Drücke von 5 bis 100 bar, vorzugsweise 5 bis 60 bar. Zur Verlangsamung der Koksabscheidung wird bei der Flüssigphasen-Isomerisierung vorzugsweise Wasserstoff zugegeben. Die bei Temperaturen oberhalb von 300°C einsetzende Transalkylierung der Chlortoluole kann durch Verdünnung mit aromatischen Kohlenwasserstoffen, wie Toluol zurückgedrängt werden. Die Belastung (WHSV = Weight-Hourly-Space-Velocity h$^{-1}$) sollte vorzugsweise zwi-schen 0,5 und 10 h$^{-1}$ liegen.

Die Isomerisierung in der Gasphase wird bei Temperaturen von 330 bis 550°C, vorzugsweise bei 330 bis 440°C, und bei Drücken von 1 bis 60 bar, vorzugsweise 5 bis 40 bar, durchgeführt. Im Gegensatz zur Flüssigphase ist bei der Gasphase die Zuspeisung von Wasserstoff in das Reaktionsgemisch unbedingt erforderlich, um eine zu rasche Deaktivierung der Katalysatoren zu vermeiden. Das Mol-Verhältnis von Wasserstoff zu Chlortoluol sollte zwischen 1 : 1 und 12 : 1 liegen. Die Belastung (WHSV) des Katalysators sollte im allgemeinen bei 1 h$^{-1}$ bis 10 h$^{-1}$ liegen. Die Regeneration der verbrauchten Katalysatoren erfolgt durch kontrolliertes Abbrennen mit sauerstoffhaltigen Gasen.

Falls man nur p-Chlortoluol herstellen will, wird Toluol vorzugsweise in Gegenwart von $FeCl_3$ und $S_2Cl_2$ bei 15—75°C, Normaldruck und einem Molverhältnis Chlor : Toluol = 0,8 bis 1,0 kernchloriert. Nach der Neutralisation, z. B. mit Kalkmilch, werden Toluol und höher chlorierte Nebenprodukte destillativ abgetrennt und so ein »gereinigtes Toluolchlorierungsprodukt« erhalten, das hier zu etwas über 50 Gew.-% aus o-Chlortoluol und zu etwa 0,3 bis 1,5 Gew.-% aus m-Chlortoluol besteht, wobei der Rest p-Chlortoluol ist.

Bei der diskontinuierlichen Arbeitsweise wird aus diesem Isomerengemisch in Stufe a) das p-Chlortoluol abgetrennt, das anschließend durch Desorption in einer Reinheit von mehr als 99% gewonnen wird. Der nichtadsorbierte Anteil an Chlortoluolisomeren besteht zu über 90 Gew.-% aus o-Chlortoluol, der Rest ist m- und p-Chlortoluol. Dieses Gemisch wird in Stufe b) dem Isomerisierungskatalysator zugeführt. Durch die Isomerisierung erhält man ein Gemisch aus etwa 48 Gew.-% o-Chlortoluol, 34 Gew.-% m-Chlortoluol und 18 Gew.-% p-Chlortoluol. Dieses Isomerengemisch wird erneut in Stufe a) behandelt, d. h. das p-Chlortoluol wird isoliert. Die nicht adsorbierten Chlortoluole werden erneut dem Isomerisierungskatalysator in Stufe b) zugeführt.

Bei der im allgemeinen bevorzugten kontinuierlichen Arbeitsweise wird aus dem gereinigten Toluol-chlorierungsprodukt in Stufe a) p-Chlortoluol isoliert, das nicht adsorbierte Isomerengemisch in Stufe b) isomerisiert und dann erneut der Stufe a) zugeführt, und zwar gemeinsam mit frischem gereinigtem Toluolchlorierungsprodukt.

Zur kontinuierlichen oder diskontinuierlichen Herstellung von p- und m-Chlortoluol wird Toluol vorzugsweise bei 15—75°C in Gegenwart von Titantetrachlorid chloriert. Das gereinigte Toluolchlorierungsprodukt (d. h. nach Neutralisation und Abtrennung von Toluol und mehrfach chlorierten Nebenprodukten) besteht zu drei Vierteln aus o-Chlortoluol, der Rest ist hauptsächlich p- neben wenig m-Isomerem.

Dieses Gemisch wird zunächst in Stufe b) mit einem Isomerisierungskatalysator behandelt. Dabei wird ein aus ca. 47 Gew.-% o-, 35 Gew.-% m- und 18 Gew.-% p-Chlortoluol bestehendes Isomerengemisch erhalten. Aus diesem Gemisch wird in Stufe a) p-Chlortoluol weitestgehend abgetrennt und mit über 99prozentiger Reinheit gewonnen. Der nicht adsorbierte Anteil an Chlortoluolen setzt sich aus ca. 58 Gew.-% o- und ca. 42 Gew.-% m-Chlortoluol zusammen. Diese binäre Mischung wird durch Destillation oder Adsorption in o- und m-Chlortoluol getrennt. Das m-Chlortoluol fällt dabei in über 99prozentiger Reinheit an. Das o-Chlortoluol wird dann — im bevorzugten kontinuierlichen Prozeß zusammen mit frischem gereinigtem Toluolchlorierungsprodukt — in Stufe b) dem Isomerisierungskatalysator zugeleitet. Aus dem nach der Isomerisierung erhaltenen Gemisch wird p-Chlortoluol erneut in Stufe a) isoliert und die verbleibenden o- und m-Isomeren wieder destillativ oder durch Adsorption getrennt. Das verbleibende o-Chlortoluol wird dann erneut in Stufe b) zurückgeführt.

Zur alleinigen Gewinnung von m-Chlortoluol geht man vor wie gerade beschrieben, mit der einzigen Ausnahme, daß das p-Chlortoluol nicht ausgeschleust, sondern in Stufe b) zurückgeführt wird.

Es ist als besonderer Vorteil des erfindungsgemäßen Verfahrens hervorzuheben, daß es erstmals möglich geworden ist, Toluol praktisch vollständig in reines p- und/oder m-Chlortoluol überzuführen. Bisher mußte bei der Produktion von p-Chlortoluol ein Zwangsanfall von o-Chlortoluol in Kauf genommen werden, für welches auf dem Markt nur ein vergleichsweise geringer Bedarf herrscht.

Nach den bisher üblichen Verfahren ist reines m-Chlortoluol nicht durch direkte Toluol-Chlorierung, sondern nur äußerst umständlich auf einem Umweg darstellbar, etwa durch Diazotierung von m-Toluidin und anschließende Reaktion mit Kupferchlorid nach Sandmayer.

Schließlich ist als weiterer Vorteil des erfindungsgemäßen Verfahrens hervorzuheben, daß, anders als bei üblichen Verfahren zur Herstellung von p-Chlortoluol, ein vergleichsweise hoher Anteil an m-Chlortoluol im Rohprodukt der Chlorierung nicht stört. Die zur Unterdrückung der Bildung von m-Chlortoluol bisher nötigen Maßnahmen, wie Verwendung von aufwendigen Katalysatorsystemen und Chlorieren bei niedrigen Temperaturen — womit zwangsläufig ein niedriger Toluol-Umsatz verbunden ist —, können somit unterbleiben, was einen nicht unerheblichen wirtschaftlichen Vorteil bedeutet. Auch die in der japanischen Patentanmeldung 3 009 723 beschriebene Oxychlorierung von Toluol mit HCl und sauerstoffhaltigen Gasen kann somit in das erfindungsgemäße Verfahren integriert werden.

Das nachfolgende Beispiel soll die Erfindung näher erläutern.

## Beispiel

### Bestimmung der Trennfaktoren verschiedener ionenausgetauschter Zeolithe für Mischungen aus p- und o- bzw. p- und m-Chlortoluol

Für die Adsorptionsversuche werden die Zeolithe unter Zusatz von 20% $Al_2O_3$ als Bindemittel in Form von Strangpreßlingen extrudiert, bei 120° C für 12 Stunden getrocknet und bei Temperaturen von 400—500° C für 4 Stunden kalziniert. Die verfestigten Strangpreßlinge werden danach zerkleinert, und für die Adsorptionsversuche wird eine Kornfraktion von 0,8—1,9 mm Durchmesser ausgesiebt. Vor den Adsorptionsversuchen werden die Zeolithe für 3 Stunden bei 450° C mit Stickstoff aktiviert. Die verwendeten analysenreinen Chlortoluole werden vor ihrem Einsatz mit einem Molsieb getrocknet. Der für die Versuche eingesetzte Adsorber, bestehend aus einem V4A-Rohr von 1000 mm Länge und 16 mm Innendurchmesser, wird elektrisch von außen beheizt. Die Isomerengemische werden mit einer Dosierpumpe über einen Verdampfer mit einer LHSV von 0,5 dem Adsorber zugeführt (LHSV = Liquid Hourly Space Velocity).

Die Adsorptionskapazität und Selektivität der Zeolithe wird mit einer äquimolaren Mischung von o- und p-Chlortoluol bei 170° C und Atmosphärendruck bestimmt. Nach Sättigung der Zeolithe mit p-Chlortoluol und Durchbruch des o-Chlortoluols wird die Dosierung unterbrochen und der Adsorber 10 Minuten mit Inertgas gespült, anschließend bei 180° C mit Benzol oder Wasserdampf desorbiert. Die Zusammensetzung von Adsorbat und Desorptiv wird gaschromatographisch ermittelt.

Zur Ermittlung des Trennvermögens der einzelnen ionenausgetauschten Zeolithe und zu einem exakten Vergleich untereinander werden ihre Trennfaktoren $\alpha_{para/ortho}$ für die äquimolare Mischung von p- und o-Chlortoluol ermittelt, und zwar nach folgender Beziehung:

$$\alpha_{para/ortho} = \frac{[para]/[ortho] \text{ in der adsorbierten Phase}}{[para]/[ortho] \text{ in der nichtadsorbierten Phase}}$$

[para] = Konzentration von p-Chlortoluol
[ortho] = Konzentration von o-Chlortoluol

**0 072 008**

In folgender Tabelle sind die Ergebnisse der unter dynamischen Bedingungen ermittelten Trennfaktoren für die untersuchten modifizierten Zeolithe zusammengefaßt:

| Zeolith | Austauschgrad | Trennfaktor $\alpha_{para/ortho}$ |
|---|---|---|
| NaX | Na$^+$ (100%) | 0,73 |
| KNaX | K$^+$ (70%) | 0,98 |
| NaY | Na$^+$ (100%) | 0,63 |
| KNaY | K$^+$ (25%) | 0,83 |
| KNaY | K$^+$ (72%) | 1,55 |
| KNaY | K$^+$ (94%) | 2,30 |
| BaNaY | Ba$^{2+}$ (76%) | 1,83 |
| KBaNaY | K$^+$, Ba$^{2+}$ (47%, 30%) | 2,35 |
| KNH$_4$NaY | K$^+$, NH$_4^+$ (77%, 17%) | 3,05 |
| K-ZSM-5 | K$^+$ (70%) | 2,85 |

Analog werden die entsprechenden Trennfaktoren für äquimolare Mischungen von p- und m-Chlortoluol ermittelt. Dabei ergeben sich gegenüber Mischungen von p- und o-Chlortoluol keine gravierenden Abweichungen der p-Chlortoluolselektivität. Die erhaltenen Werte sind in nachstehender Tabelle zusammengestellt:

| Zeolith | Austauschgrad | Trennfaktor $\alpha_{para/meta}$ |
|---|---|---|
| KNaY | K$^+$ (25%) | 0,90 |
| KNaY | K$^+$ (94%) | 2,27 |
| KBaNaY | K$^+$, Ba$^{2+}$ (47%, 30%) | 2,29 |
| KNH$_4$NaY | K$^+$, NH$_4^+$ (50%, 43%) | 2,47 |
| KNH$_4$NaY | K$^+$, NH$_4^+$ (77%, 17%) | 3,15 |
| K-ZSM-5 | K$^+$ (70%) | 3,35 |

Adsorptive Trennung des p-Chlortoluols von o- und m-Chlortoluol

Über einen durch 30minütiges Precoking mit Toluol bei 600° C modifizierten K-ZSM-5-Zeolithen wird ein Isomerengemisch, bestehend aus 52,6 Gew.-% o-Chlortoluol, 34,5 Gew.-% m-Chlortoluol und 12,9 Gew.-% p-Chlortoluol geleitet. Bei einer WHSV von 0,2 h$^{-1}$, einer Temperatur von 180° C und Normaldruck wird unter stationären Bedingungen am Ausgang des obenerwähnten Adsorbers eine Isomerenverteilung von 59,9 Gew.-% o-Chlortoluol, 39,3 Gew.-% m-Chlortoluol und 0,79 Gew.-% p-Chlortoluol erhalten.

5

## Kontinuierliche Abtrennung von p-Chlortoluol

Ein durch Chlorierung von Toluol bei 50°C in Gegenwart von FeCl₃ erhaltenes Reaktionsgemisch wird von nicht umgesetztem Toluol und höher chlorierten Nebenprodukten destillativ befreit. Das so erhaltene »gereinigte Toluolchlorierungsprodukt« besteht aus 64 Gew.-% o-Chlortoluol, 2,6 Gew.-% m-Chlortoluol und 33,3 Gew.-% p-Chlortoluol. Dieses Gemisch wird durch einen Festbettadsorber geleitet, der einen mit Kalium modifizierten Y-Zeolithen enthält. Dabei wird das p-Chlortoluol bei 200°C und 5 bar selektiv adsorbiert.

Das den Adsorber verlassende Gemisch wird bei 220°C und 30 bar durch einen Rohrreaktor geleitet, der als Isomerisierungskatalysator einen dealuminierten H-Mordenit enthält. Dabei wird das Gemisch isomerisiert. Die den Reaktor verlassende Mischung wird zu erneuten Adsorption des p-Chlortoluols zum Adsorber zurückgeführt. Sie wird gemeinsam mit frischem gereinigtem Toluolchlorierungsprodukt durch den Adsorber geleitet.

Im stationären Betrieb enthält das in den Adsorber eintretende Gemisch 51,8 Gew.-% o-Chlortoluol, 29,1 Gew.-% m-Chlortoluol und 19,2 Gew.-% p-Chlortoluol. Die Adsorption erfolgt mit einer WHSV von 0,3 h⁻¹. Die den Adsorber verlassende Mischung enthält 63,2 Gew.-% o-Chlortoluol, 35,6 Gew.-% m-Chlortoluol und 1,2 Gew.-% p-Chlortoluol. Diese Mischung wird im Isomerisierungsreaktor mit einer WHSV von 2 h⁻¹ in ein Gemisch aus 48,9 Gew.-% o-Chlortoluol, 35 Gew.-% m-Chlortoluol und 16,1 Gew.-% p-Chlortoluol umgewandelt, welches, wie gesagt, zum Adsorber zurückgeführt wird.

## Patentansprüche

1. Verfahren zur Herstellung von p-Chlortoluol und/oder m-Chlortoluol durch Kernchlorierung von Toluol, gekennzeichnet durch Verwendung der beiden folgenden Stufen a) und b) bei der Weiterverarbeitung des aus einem Isomerengemisch bestehenden Toluol-Chlorierungsproduktes:

a) Isolation von p-Chlortoluol aus einem Gemisch von o-, m- und p-Chlortoluol durch selektive Adsorption des p-Chlortoluols an einem mittel- oder großporigen Zeolith und anschließende Desorption des p-Chlortoluols,

b) Behandlung eines an o-Chlortoluol reichen Isomerengemisches mit einem Zeolith vom Pentasil-, Mordenit- oder Faujasit-Typ als Isomerisierungskatalysator.

wobei man zur
ausschließlichen Herstellung von p-Chlortoluol zunächst aus dem Toluol-Chlorierungsprodukt in Stufe a) p-Chlortoluol isoliert und ausschleust, dann das verbleibende Isomerengemisch in Stufe b) behandelt, um seinen Gehalt an m- und p-Isomeren zu erhöhen und dann in Stufe a) zurückführt, und zur Herstellung von m-Chlortoluol, und gegebenenfalls zusätzlich von p-Chlortoluol, zunächst das Toluol-Chlorierungsprodukt in Stufe b) behandelt, um seinen Gehalt an m- und gegebenenfalls an p-Isomerem zu erhöhen, dann in Stufe a) p-Chlortoluol isoliert und dieses in Stufe b) zurückführt oder gegebenenfalls ausschleust und das verbleibende, im wesentlichen aus o- und m-Isomeren bestehende Gemisch destillativ oder adsorptiv trennt und dann das o-Chlortoluol in Stufe b) zurückführt und das m-Chlortoluol ausschleust.

2. Verfahren nach Anspruch 1, dadurch gekennzeichnet, daß man als Zeolith in Stufe a) ein modifiziertes Pentasil oder einen modifizierten X- oder Y-Zeolith verwendet.

## Claims

1. A process for preparing p-chlorotoluene and/or m-chlorotoluene by ring-chlorination of toluene, which comprises using the following two stages a) and b) when further processing the toluene chlorination product composed of mixed isomers:

a) Isolation of p-chlorotoluene from a mixture of o-, m- and p-chlorotoluene by selective adsorption of p-chlorotoluene onto a mesoporous or macroporous zeolite and subsequent desorption of p-chlorotoluene.

b) Treatment of an o-chlorotoluene-rich mixture of isomers with a zeolite of the pentasil, mordenite or faujasite type as isomerization catalyst

wherein for
the exclusive preparation of p-chlorotoluene first p-chlorotoluene is isolated and removed from the toluene chlorination product in stage a) and the remaining mixed isomers are then treated in stage b) to increase their content of m- and p-isomers and then returned into stage a) and
for the preparation of m-chlorotoluene and, if appropriate, additionally of p-chlorotoluene first the toluene chlorination product is treated in stage b) to increase its content of m- and, if appropriate,

# 0 072 008

p-isomer, p-chlorotoluene is then isolated in stage a) and returned to stage b), or, if appropriate, removed, and the remaining mixture essentially comprising o- and m-isomers is separated by distillation or adsorption and o-chlorotoluene is then returned into stage b) and m-chlorotoluene is removed.

2. The process as claimed in claim 1, wherein the zeolite used in stage a) is a modified pentasil or a modified X or Y zeolite.

## Revendications

1. Procédé pour préparer du p-chloro-toluène et/ou du m-chloro-toluène par chloration du toluène sur son noyau, procédé caractérisé en ce que, lors du traitement ultérieur du produit de chloration du toluène, qui est constitué d'un mélange d'isomères, on passe par les deux étapes a) et b) suivantes:

a)   isolement du p-chloro-toluène à partir d'un mélange d'ortho, de méta et de para-chloro-toluènes par adsorption sélective du p-chloro-toluène sur un zéolite à pores moyens ou gros, puis désorption du p-chloro-toluène,

b)   traitement d'un mélange d'isomères riche en o-chloro-toluène par un catalyseur d'isomérisation qui est un zéolite du type du Pentasil, de la mordénite ou de la faujasite,

plus précisément:
si l'on veut préparer exclusivement le p-chloro-toluène on isole d'abord, dans l'étape a), le p-chloro-toluène du produit de chloration du toluène et on le fait sortir, puis on traite le mélange d'isomères restant dans l'étape b) pour augmenter sa teneur en isomères méta et para et ensuite on le renvoie à l'étape a), et
si l'on veut préparer le m-chloro-toluène et éventuellement, en plus, du p-chloro-toluène on traite d'abord le produit de chloration du toluène dans l'étape b) pour augmenter sa teneur en l'isomère méta et éventuellement en l'isomère para, puis on isole le p-chloro-toluène dans l'étape a) et on le renvoie à l'étape b), ou éventuellement on le fait sortir, et on sépare par distillation ou adsorption le mélange restant, qui est essentiellement constitué des isomères ortho et méta, puis on renvoie l'o-chloro-toluène à l'étape b) et on fait sortir le m-chloro-toluène.

2. Procédé selon la revendication 1, caractérisé en ce qu'on utilise comme zéolite, dans l'étape a), un Pentasil modifié ou un zéolite X ou Y modifié.